# EUROPEAN PATENT APPLICATION

(11) **EP 2 156 848 A1**
(43) Date of publication of application: **24.02.2010**
(21) Application number: 08740264.0
(22) Date of filing: 11.04.2008
(51) Int. Cl.: A61K 47/34, A61K 9/14, A61K 45/00, A61K 47/02, A61K 47/18, A61K 47/22, A61K 47/24

(54) **LOW-MOLECULE DRUG-CONTAINING NANOPARTICLE HAVING SUSTAINED RELEASE NEGATIVELY CHARGED GROUP**

(30) Priority: 14.05.2007 JP 2007128059
(71) Applicant: LTT Bio-Pharma Co., Ltd., Minato-ku, Tokyo 1048315 (JP)
(72) Inventor: ISHIHARA, Tsutomu, Tokyo 143-0024 (JP); MIZUSHIMA, Yutaka, Tokyo 106-0032 (JP); MIZUSHIMA, Toru, Kumamoto-shi Kumamoto 862-0949 (JP)
(74) Representative: Albrecht, Thomas
(86) International application number: PCT/JP2008/057167
(87) International publication number: WO 2008/139804

(57) **Abstract**

A nanoparticle containing a low-molecular-weight drug having a negatively charged group is provided that is effectively targeted to an affected site, is capable of sufficiently sustained release of the drug, and has a reduced tendency to accumulate in the liver to cause reduced side effects. The nanoparticle containing a low-molecular-weight drug having a negatively charged group is obtained by hydrophobicizing the low-molecular-weight drug having a negatively charged group with a metal ion, and reacting the hydrophobicized drug with poly L-lactic acid or poly (L-lactic acid/glycolic acid) copolymer and poly DL- or L-lactic acid-polyethylene glycol block copolymer or poly(DL- or L-lactic acid/glycolic acid)-polyethylene glycol block copolymer.

## Description

### TECHNICAL FIELD

The present invention relates to a drug-containing nanoparticle and, more particularly, to a nanoparticle containing a low-molecular-weight drug having a negatively charged group. The nanoparticle is effectively targeted to an affected site, is capable of sufficiently sustained release of the drug, has a reduced tendency to accumulate in the liver, and has an improved long-circulating property.

### BACKGROUND ART

Many studies have been conducted in an attempt to encapsulate drugs into microparticles or nanoparticles of lactic acid-glycolic acid copolymer (which may be referred to as "PLGA," hereinafter) or polylactic acid (which may be referred to as "PLA," hereinafter).

For example, US Patent No. 4,652,441 (Patent Document 1) discloses PLGA or other microcapsules containing biologically active polypeptides and a method for producing such microcapsules. Japanese Translation of PCT International Application No. Hei. 10-511957 (Patent Document 2) discloses PLGA or other nanoparticle containing different drugs for intravascular administration. Japanese Patent Application Laid-Open No. Hei. 8-217691 (Patent Document 3) discloses a sustained release preparation formed of PLGA or other microcapsules encapsulating water-insoluble or poorly water-soluble polyvalent, metal salts of physiologically active water-soluble peptide materials.

However, none of these prior art patents contain any description about converting a low-molecular-weight drug having a negatively charged group to hydrophobic one with a metal ion, and encapsulating the drug into a PLA-PEG block copolymer or PLGA-PEG block copolymer including PLA or PLGA bound to polyethylene glycol (which may be referred to as "PEG," hereinafter).

International Patent Publication No. WO 2003/101493 (Patent Document 4), a patent application of which was filed by the applicant of the present application, describes a preparation obtained by encapsulating a drug into PLGA or PLA fine particles, and having a surfactant adsorb onto the surface of the fine particles. International Patent Publication No. WO 2004/84871 (Patent Document 5), a patent application of which was also filed by the applicant of the present application, describes a preparation obtained by encapsulating a low-molecular-weight pharmaceutical product, having a negatively charged group and hydrophobicized by a metal ion, into PLGA or PLA nanoparticles, and having a surfactant adsorb onto the surface of the nanoparticles.

However, the fine particles disclosed in Patent Document 4 can encapsulate only small fractions of drugs and also tend to burst at an early stage, resulting in insufficiently sustained release of drugs. In comparison, the nanoparticles disclosed in Patent Document 5 can encapsulate increased fractions of drugs and are less likely to burst at an early stage. However, when administered intravenously, these particles tend to undesirably remain in the liver.

With regard to block copolymers composed of polylactic acid or poly(lactic acid/glycolic acid) copolymer and polyethylene glycol, Japanese Patent Application Laid-Open No. Sho. 58-191714 (Patent Document 6) describes a block copolymer of polyethylene glycol, a hydrophilic polymer, and polylactic acid, a hydrophobic polymer. Also, Japanese Patent Application Laid-Open No. Hei. 9-157368 (Patent Document 7) describes a method for purifying a triblock copolymer composed of polylactic acid-polyethylene glycol-polylactic acid.

Block copolymers composed of polylactic acid or poly(lactic acid/glycolic acid) copolymer and polyethylene glycol may be used as a material to form a pharmaceutical composition. One example is a pharmaceutical composition described in Japanese Patent Application Laid-Open No. Hei. 2-78629 (Patent Document 8) that contains a copolymer of lactic acid and/or glycolic acid with polyethylene glycol, along with a polypeptide. Another example is a solution described in Japanese Patent Application Laid-Open No. Hei. 9-151136 (Patent Document 9) that contains a polylactic acid-polyethylene glycol copolymer and a protein.

Nonetheless, no one has ever described nanoparticles containing a low-molecular-weight drug having a negatively charged group that are obtained by hydrophobicizing a low-molecular-weight drug having a negatively charged group with a metal ion, and reacting the hydrophobicized drug with a polylactic acid-polyethylene glycol block copolymer or a poly(lactic acid/glycolic acid)-polyethylene glycol block copolymer.

The applicant of the present application has previously filed a patent application claiming a drug-containing nanoparticle (International Patent Application No. PCT/JP2006/323820). The nanoparticle is obtained by hydrophobicizing a low-molecular-weight drug having a negatively charged group with a metal ion, and encapsulating the hydrophobicized drug into a nanoparticle obtained by reacting polylactic acid-polyethylene glycol block copolymer or poly(lactic acid/glycolic acid)-polyethylene glycol block copolymer with polylactic acid or poly(lactic acid/glycolic acid) copolymer. The nanoparticle is effectively targeted to an affected site, is capable of sufficiently sustained release of the drug, has a reduced tendency to accumulate in the liver, and has an improved long-circulating property.

Patent Document 1: US Patent No. 4,652,441
Patent Document 2: Japanese Translation of PCT International Application No. Hei. 10-5119957
Patent Document 3: Japanese Patent Application Laid-Open No. Hei. 8-217691
Patent Document 4: International Patent Publication No. WO 2003/101493
Patent Document 5: International Patent Publication No. WO 2004/84871
Patent Document 6: Japanese Patent Application Laid-Open No. Sho. 58-191714
Patent Document 7: Japanese Patent Application Laid-Open No. Hei. 9-157368
Patent Document 8: Japanese Patent Application Laid-Open No. Hei. 2-78629
Patent Document 9: Japanese Patent Application Laid-Open No. Hei. 9-151136

### SUMMARY OF THE INVENTION

### PROBLEMS TO BE SOLVED BY THE INVENTION

The present invention is an improvement of the invention claimed in PCT/JP2006/323820. Thus, it is an object of the present invention to provide a nanoparticle containing a low-molecular-weight drug having a negatively charged group that can effectively target the low-molecular-weight drug having a negatively charged group to an affected site, is capable of sufficiently sustained release of the drug, and has a reduced tendency to accumulate in the liver to cause reduced side effects, and that is in particular capable of more effective sustained release than the nanoparticle described in PCT/JP2006/323820.

The present inventors have put a significant amount of effort into achieving the above-described object and, as a result, have found that a particular nanoparticle containing a low-molecular-weight drag having a negatively charged group is effectively targeted to an affected site, is capable of sufficiently sustained release of the drug, has a reduced tendency to accumulate in the liver, and has an improved long-circulating property. Of these properties, the nanoparticle has particularly favorable sustained release properties. Specifically, such a nanoparticle can be obtained by hydrophobicizing a low-molecular-weight drug having a negatively charged group with a metal ion, and reacting the hydrophobicized drug with poly L-lactic acid or poly(L-lactic acid/glycolic acid) copolymer and poly DL- or L-lactic acid-polyethylene glycol block copolymer or poly(DL- or L-lactic acid/glycolic acid)-polyethylene glycol block copolymer. This finding ultimately led to the present invention.
In particular, the present invention has a feature in that it can achieve particularly favorable sustained release of the drug by using poly L-lactic acid or poly(L-lactic acid/glycolic acid) copolymer, each an L-form, rather than poly DL-lactic acid or poly(DL-lactic acid/glycolic acid) copolymer.

### MEANS FOR SOLVING THE PROBLEMS

Accordingly, the present invention provides a nanoparticle containing a low-molecular-weight drug having a negatively charged group that is effectively targeted to an affected site, is capable of sufficiently sustained release of the drug, has a reduced tendency to accumulate in the liver to cause reduced side effects, and has an improved long-circulating property.
The present invention further provides an intravenous preparation, a local injection preparation, a nasal preparation, an ophthalmic preparation, an inhalation preparation, a spray preparation and other parenteral preparations that contain the nanoparticle as an active ingredient.

More specifically, the present invention includes the following configurations:
(1) A nanoparticle containing a low-molecular-weight drug having a negatively charged group, the nanoparticle obtained by hydrophobicizing a low-molecular-weight drug having a negatively charged group with a metal ion, and reacting the hydrophobicized drug with poly L-lactic acid or poly(L-lactic acid/glycolic acid) copolymer and poly DL- or L-lactic acid-polyethylene glycol block copolymer or poly(DL- or L-lactic acid/glycolic acid)-polyethylene glycol block copolymer;
(2) The nanoparticle containing a low-molecular-weight drug having a negatively charged group according to (1) above, further containing a basic low-molecular-weight compound mixed therewith;
(3) The nanoparticle containing a low-molecular-weight drug having a negatively charged group according to (1) or (2) above, further containing a surfactant compounded thereinto;
(4) The nanoparticle containing a low-molecular-weight drug having a negatively charged group according to any of (1) to (3) above, having a particle size of 20 to 300 nm, and preferably 50 to 200 nm;
(5) The nanoparticle containing a low-molecular-weight drug having a negatively charged group according to (1) or (2) above, wherein the metal ion is one or two or more of zinc ion, iron ion, copper ion, nickel ion, beryllium ion, manganese ion or cobalt ion;
(6) The nanoparticle containing a low-molecular-weight drug having a negatively charged group according to (1) or (2) above, wherein the low-molecular-weight drug having the negatively charged group has a phosphate group, a sulfate group or a carboxyl group due to the hydrophobicization with the metal ion;
(7) The nanoparticle containing a low-molecular-weight drug having a negatively charged group according to (1), (2) or (6) above, wherein the low-molecular-weight drug having the negatively charged group is an anti-inflammatory steroid, a non-steroid anti-inflammatory agent, prostaglandin or a derivative thereof, an antimicrobial agent or an anticancer agent;
(8) The nanoparticle containing a low-molecular-weight drug having a negatively charged group according to (1) or (2) above, wherein the poly DL- or L-lactic acid-polyethylene glycol block copolymer or poly(DL- or L-lactic acid/glycolic acid)-polyethylene glycol block copolymer has a weight average molecular weight of 3,000 to 30,000;
(9) The nanoparticle containing a low-molecular-weight drug having a negatively charged group according to (2) above, wherein the basic low-molecular-weight compound is one or two or more selected from (dimethylamino)pyridine, pyridine, piperidine, pyrimidine, pyrazine, pyridazine, quinoline, quinuclidine, isoquinoline, bis(dimethylamino)naphthalene, naphthylamine, morpholine, amantadine, aniline, spermine, spermidine, hexamethylenediamine, putrescine, cadaverine, phenetylamine, histamine, diazabicyclooctane, diisopropylethylamine, monoethanolamine, diethanolamine, triethanolamine, ethylamine, diethylamine, triethylamine, methylamine, dimethylamine, trimethylamine, triethylenediamine, diethylenetriamine, ethylenediamine and trimethylenediamine;
(10) The nanoparticle containing a low-molecular-weight drug having a negatively charged group according to (3) above, wherein the surfactant is one or two or more selected from phosphatidylcholine, polyoxyethylene (20) sorbitan monooleate, polyoxyethylene (20) sorbitan monolaurate, polyoxyethylene (20) sorbitan monostearate, polyoxyethylene (20) sorbitan monopalmitate, polyoxyethylene (20) sorbitan trioleate, polyoxyethylene (80) octylphenyl ether, polyoxyethylene (20) cholesterol ester, lipid-polyethylene glycol, polyoxyethylene hydrogenated castor oil and fatty acid-polyethylene glycol copolymer;
(11) A parenteral preparation containing as an active ingredient the nanoparticle containing a low-molecular-weight drug having a negatively charged group according to (1) to (10) above;
(12) The parenteral preparation according to (11) above, wherein the preparation is an intravenous preparation, a local injection preparation, a nasal preparation, an ophthalmic preparation, an inhalation preparation or a spray preparation;
(13) A method for producing the nanoparticles containing a low-molecular-weight drug having a negatively charged group according to (1) above, the method including:
   mixing the low-mclecular-weight drug having the negatively charged group with the metal ion in a solvent to obtain the hydrophobicized drug; and
   mixing with the resulting mixture the poly L-lactic acid or the poly(L-lactic acid/glycolic acid) copolymer and the poly DL- or L-lactic acid-polyethylene glycol block copolymer or the poly(DL- or L-lactic acid/glycolic acid)-polyethylene glycol block copolymer;
(14) The method according to (13) above for producing the nanoparticle containing a low-molecular-weight drug having a negatively charged group, the method further including mixing a basic low-molecular-weight compound therewith;
(15) The method according to (14) above for producing the nanoparticle containing a low-molecular-weight drug having a negatively charged group, wherein the basic low-molecular-weight compound is one or two or more selected from (dimethylamino)pyridine, pyridine, piperidine, pyrimidine, pyrazine, pyridazine, quinoline, quinuclidine, isoquinoline, bis(dimethylamino)naphthalene, naphthylamine, morpholine, amantadine, aniline, spermine, spermidine, hexamethylenediamine, putrescine, cadaverine, phenetylamine, histamine, diazabicyclooctane, diisopropylethylamine, monoethanolamine, diethanolamine, triethanolamine, ethylamine, diethylamine, triethylamine, methylamine, dimethylamine, trimethylamine, triethylenediamine, diethylenetriamine, ethylenediamine and trimethylenediamine.

In brief, the present invention has a feature in that a nanoparticle encapsulating a low-molecular-weight drug having a negatively charged group is produced by hydrophobicizing the low-molecular-weight drug having a negatively charged group with a metal ion, and reacting the hydrophobicized drug with poly L-lactic acid or poly(L-lactic acid/glycolic acid) copolymer and poly DL- or L-lactic acid-polyethylene glycol block copolymer or poly(DL- or L-lactic acid/glycolic acid)-polyethylene glycol block copolymer.

### EFFECTS OF THE INVENTION

The nannoparticle containing a low-molecular-weioht drug having a negatively charged group provided in accordance with the present invention can target the low-molecular-weight drug having a negatively charged group to an affected site, is capable of sufficiently sustained release of the drug, has a reduced tendency to accumulate in the Liver to cause reduced side effects, and also has an improved long-circulating property. In particular, the nanoparticle is capable of effective sustained release of the drug. What is the feature of the present invention is that it can achieve particularly favorable sustained release of the drug when poly L-lactic acid or poly(L-lactic acid/glycolic acid) copolymer, each an L-form, is used instead of poly DL-lactic acid or poly(DL-lactic acid/glycolic acid) copolymer.
Accordingly, the present invention has made it possible to target a low-molecular-weight drug having a negatively charged group and has achieved improved sustained release of such a drug while reducing side effects caused by the accumulation of the drug in the liver and achieving an improved long-circulating property, each of which is a task that has never been achieved to a sufficient level.

### BRIEF DESCRIPTION OF THE DRAWINGS

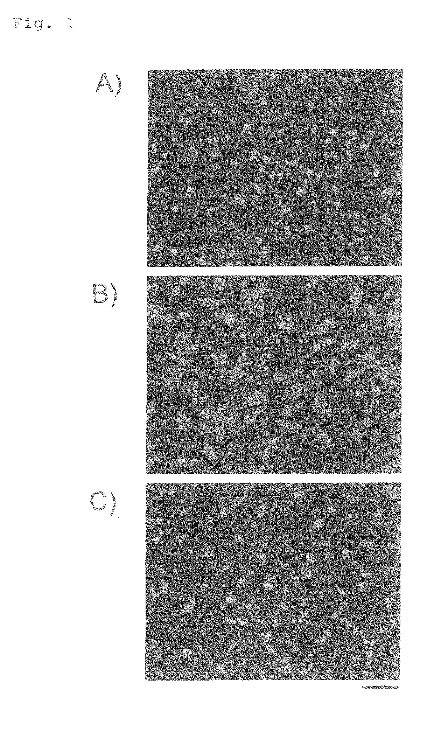

Fig. 1 shows fluorescence micrographs of cells incorporating nanoparticles encapsulating rhodamine according to Example 4.
The figure shows the density distribution for cells with rhodamine alone (Picture A)), with nanoparticles made from PLLA alone encapsulating rhodamine and PGE₁ (Picture B)), and with nanoparticles made from PLLA and PDLLA-PEG encapsulating rhodamine and PGE₁ (Picture C)). The scale bar indicates 50 µm.

### BEST MODE FOR CARRYING OUT THE INVENTION

The nanoparticle containing a low-molecular-weight drug having a negatively charged group, which form one aspect of the present invention, is obtained by hydrophobicizing a low-molecular-weight drug having a negatively charged group with a metal ion, and reacting the hydrophobicized drug with poly L-lactic acid or poly (L-lactic acid/glycolic acid) copolymer and poly DL- or L-lactic acid-polyethylene glycol block copolymer or poly(DL- or L-lactic acid/glycolic acid)-polyethylene glycol block copolymer. When necessary, a surfactant may be compounded to stabilize the resulting nanoparticles.

One feature of the nanoparticle of the present invention containing a low-molecular-weight drug having a negatively charged group is the use of poly L-lactic acid or poly(L-lactic acid/glycolic acid) copolymer, each an L-isomer, as a biodegradable polymer to form the nanoparticles.
Poly-L-lactic acid is known to have a different solubility in an organic solvent and higher crystallinity than poly-DL-lactic acid. In the present invention, poly-L-lactic acid is mixed with poly DL- or L-lactic acid-polyethylene glycol block copolymer or poly(DL- or L-lactic acid/glycolic acid)-polyethylene glycol block copolymer to form the nanoparticle. In this manner, the crystallization of poly-L-lactic acid in the aqueous phase can be prevented, so that the resulting nanoparticle can be dispersed to form a stable solution.
Since poly-L-lactic acid is insoluble to acetone, a mixture of acetone/dioxane or acetone/tetrahydrofuran is used to increase the solubility of the poly-L-lactic acid to form the nanoparticle.

The nanoparticle containing a low-molecular-weight drug having a negatively charged group may contain a surfactant compounded thereto to stabilize the resulting nanoparticle and thus prevent the aggregation of the particles.

The nanoparticle of the present invention containing a low-molecular-weight drug having a negatively charged group provided in the above-described manner can be administered in the form of a parenteral preparation, such as an intravenous preparation, a local injection preparation, a nasal preparation, an ophthalmic preparation, an inhalation preparation and a spray preparation.

The nanoparticle according to the present invention, containing a low-molecular-weight drug having a negatively charged group can be produced in the following manner,
A low-molecular-weight drug having a negatively charged group is converted to hydrophobic drug by mixing with a metal ion in an organic solvent or an aqueous organic solvent, and to this mixture, poly L-lactic acid or a poly(L-lactic acid/glycolic acid) copolymer and a poly DL- or L-lactic acid-polyethylene glycol block copolymer or a poly(DL- or L-lactic acid/glycolic acid)-polyethylene glycol, block copolymer is added and the mixture is stirred. The resulting mixture is then dispersed in water to form the desired nanoparticle.

Alternatively, a solution obtained by dissolving poly L-lactic acid or poly(L-lactic acid/glycolic acid) copolymer and poly DL- or L-lactic acid-polyethylene glycol block copolymer or poly(DL- or L-lactic acid/glycolic acid)-polyethylene glycol block copolymer in a solvent, an aqueous solution of a low-molecular-weight drug having a negatively charged group, and an aqueous solution of a metal ion may be added and mixed together at once to form the nanoparticle.

By using the poly DL- or L-lactic acid-polyethylene glycol block copolymer or the poly(DL- or L-lactic acid/glycolic acid)-polyethylene glycol block copolymer as a surface-modifying agent of the nanoparticle, the crystallization of poly L-lactic acid or poly(L-lactic acid/glycolic acid) copolymer can be prevented, so that the resulting nanoparticle will have a uniform size and will become stable.

The used metal ion may be any of zinc ion, iron ion, copper ion, nickel ion, beryllium ion, manganese ion and cobalt ion. One or two or more of these ions are used in the form of water-soluble metal salts. Of these ions, zinc ion and iron ion are preferably used in the form of zinc chloride and iron chloride, respectively.

The solvent used in the above-described reaction is preferably an organic solvent, such as acetone, acetonitrile, ethanol, methanol, propanol, dimethylformamide, dimethylsulfoxide, dioxane and tetrahydrofuran, or an aqueous solvent thereof. Of these solvents, acetone, dimethylformamide, dioxane and tetrahydrofuran are particularly preferred.

The low-molecular-weight drug having a negatively charged group preferably includes in its molecule a phosphate group, a sulfate group or a carboxyl group that binds to the metal ion to facilitate the hydrophobicization of the drug. Also, the drug preferably has a molecular weight of 1,000 or less.

While a variety of low-molecular-weight drugs having a negatively charged group may be used in the present invention, water-soluble anti-inflammatory steroids, nonsteroidal anti-inflammatory agents, prostaglandin or derivatives thereof, antimicrobial agents or anticancer agents are preferably used. Specific examples include, but are not limited to, anti-inflammatory steroids, such as betamethasone phosphate, dexamethasone phosphate, prednisolone phosphate, hydrocortisone phosphate, prednisolone succinate and hydrocortisone succinate; nonsteroidal anti-inflammatory agents, such as loxoprofen, ibuprofen, ketoprofen, diclofenac and fenbufen; prostaglandin E₁, prostaglandin E₂ and derivatives thereof; antimicrobial agents, such as vancomycin, chloramphenicol succinate, latamoxef, cefpirome, clindamycin phosphate and carumonam; and anticancer agents, such as vincristine and vinblastine.

Although the poly DL- or L-lactic acid-polyethylene glycol block copolymer (the DL-form may be referred to as "PDLLA-PEG" and the L-form may be referred to as "PLLA-PEG") or the poly(DL- or L-lactic acid/glycolic acid)-polyethylene glycol block copolymer (the DL-form may be referred to as "PDLLGA-PEG" and the L-form may be referred to as "PLLGA-PEG") can be produced by reacting poly DL-lactic acid (which may be referred to as "PDLLA") or poly L-lactic acid (which may be referred to as "PLLA"), or poly (DL-lactic acid/glycolic acid) copolymer (which may be referred to as "PDLLGA") or poly(L-lactic acid/glycolic acid) copolymer (which may be referred to as "PLLGA") (there polymers will be referred to as "block A") with polyethylene glycol (which may be referred to also as "PEG") ("block B") in the presence of a condensation agent, such as ethylene dimethylaminopropyl carbodiimide, similar commercially available block copolymers may also be used.

To achieve the object of the present invention, the block copolymer may be any of the following types: A-B, A-B-A and B-A-B. These block copolymers preferably have a weight average molecular weight of 3,000 to 30,000.

The nanoparticle containing a low-molecular-weight drug having a negatively charged group of the present invention tends to become larger in size and encapsulate more drugs in the nanoparticles as the mixing ratio of the poly L-lactic acid or the poly (L-lactic acid/glycol acid) copolymer to the poly DL- or L-lactic acid-polyethylene glycol block copolymer or the poly(DL- or L-lactic acid/glycolic acid)-polyethylene glycol block copolymer is increased.

A basic low-molecular-weight compound may also be mixed to the nanoparticle of the present invention containing a low-molecular-weight drug having a negatively charged group to increase the encapsulation efficiency (the amount of the drug that can be encapsulated by the nanoparticle) up to about 10%.
Examples of such basic low-molecular weight compounds include (dimethylamino)pyridine, pyridine, piperidine, pyrimidine, pyrazine, pyridazine, quinoline, quinuclidine, isoquinoline, bis(dimethylamino)naphthalene, naphthylamine, morpholine, amantadine, aniline, spermine, spermidine, hexamethylenediamine, putrescine, cadaverine, phenetylamine, histamine, diazabicyclooctane, diisopropylethylamine, monoethanolamine, diethanolamine, triethanolamine, ethylamine, diethylamine, triethylamine, methylamine, dimethylamine, trimethylamine, triethylenediamine, diethylenetriamine, ethylenediamine and trimethylenediamine. Of these compounds, secondary and tertiary amines are preferred with diethanolamine being particularly preferred.

A surfactant may then be compounded to the resulting nanoparticle containing a low-molecular-weight drug having a negatively charged group to stabilize the nanoparticle and prevent the aggregation of the particles. This makes the nanoparticle suitable for making a preparation containing the nanoparticles.

Examples of the surfactant that can be used include phosphatidylcholine, polyoxyethylene (20) sorbitan monooleate, polyoxyethylene (20) sorbitan monolaurate, polyoxyethylene (20) sorbitan monostearate, polyoxyethylene (20) sorbitan monopalmitate, polyoxyethylene (20) sorbitan trioleate, polyoxyethylene (80) octylphenyl ether, polyoxyethylene (20) cholesterol ester, lipid-polyethylene glycol, polyoxyethylene hydrogenated castor oil, and fatty acid-polyethylene glycol copolymer. One or two or more selected from these surfactants are preferably used.

The nanoparticle containing a low-molecular-weight drug having a negatively charged group provided in accordance with the present invention typically has a particle size in the range of 20 to 300 nm, and preferably in the range of 50 to 200 nm. The particle size can be determined depending on the type of the target site to which a particular drug is targeted.
For example, when the drug is intended to treat diseases such as arthritis, cancer and arteriosclerosis obliterans, the nanoparticles having a particle size of 50 to 200 nm are preferably injected intravenously. The particle size can be adjusted by varying the amount of the solvent to dissolve the PDLLA-PEG or PLLA-PEG, or PDLLGA-PEG or PLLGA-PEG, which is preferably acetone or dioxane. Increasing the amount of acetone or dioxane leads to a decreased particle size of the nanoparticles. The nanoparticle having a larger particle size tends to achieve a higher encapsulation efficiency of the drug.

The nanoparticles of the present invention containing a low-molecular-weight drug having a negatively charged group prepared in the above-described manner are separated from the solution or the suspension containing the nanoparticles by a suitable purification technique, such as centrifugation, ultrafiltration, gel filtration, filtration by filter and fiber dialysis. The separated nanoparticles are freeze-dried and stored.

A stabilizer and/or a disperser are preferably added to the separated nanoparticles for freeze-drying so that the freeze-dried preparation can be re-suspended prior to administ-ration. Preferred examples of such stabilizers and dispersers include sucrose, trehalose and carboxymethylcellulose sodium.

The nanoparticle containing a low-molecular-weight drug having a negatively charged group provided in accordance with the present invention can be used in pharmaceutical products provided in the form of parenteral preparations such as intravenous preparations, local injection preparations, nasal preparations, ophthalmic preparations, inhalation preparations and spray preparations. The characteristics and advantages of the nanoparticle of the present invention can be most effectively exploited when they are prepared as an intravenous preparation.

Pharmaceutically acceptable bases and other additives are also used in preparing the above-described parenteral preparations. Specific example of the base and the additive include physiological saline; sugars, such as monosaccharides, disaccharides, sugar alcohols and polysaccharides; polymer additives, such as hydroxyethylcellulose, hydroxypropylcellulose and methylcellulose; and ionic or nonionic surfactants. These bases and the additives are properly selected depending on the dosage form.

### EXAMPLES

The present invention will now be described in further detail with reference to examples, which are not intended to limit the scope of the invention in any way.

### Example 1: Synthesis of poly DL-lactic acid-polyethylene glycol copolymer (PDLLA-PEG)

40 g of methoxy-PEG (Mw 5200, manufactured by NOF Corporation), 40 g of D,L-lactide (manufactured by Purac), tin octylate (400 mg) were placed in a two-neck round-bottom flask and thoroughly mixed. The mixture was degassed by a hydraulic pump and was then heated in an oil bath at 110°C to dissolve the mixture. Once dissolved, the temperature was raised to 155°C and the reaction was carried out for 4 hours. Subsequently, the reaction product (solid) was cooled and dissolved in approx. 250 mL of dichloromethane. The solution was then precipitated for purification by slowly adding it to 2.5 L of ice-cooled isopropanol and the precipitate was freeze-dried to obtain poly DL-lactic acid-polyethylene glycol block copolymer (PDLLA-PEG). The product was evaluated by gel permeation chromatography (GPC) or proton NMR.

The results of GPC analysis indicated an increase in the molecular weight as compared to methoxy-PEG. Also, the results of proton NMR indicated the presence of poly lactic acid. These observations together suggested that the synthesized product was PDLLA-PEG. The same procedure was performed using a different amount of D,L-lactide to obtain PDLLA-PEG having a different molecular weight.
PDLLGA-PEG was also synthesized by performing the same procedure except that the D,L-lactide was replaced with a mixture of D,L-lactide and glycolide.

### Example 2: Production of nanoparticles formed of PLLA and PDLLA-PEG and encapsulating prostaglandin E₁ (PGE₁)

A predetermined amount of PLLA (manufactured by Taki Chemical Co., Ltd.) was weighed and dissolved in 225 µL of dioxane. A predetermined amount of PDLLA-PEG synthesized in Example 1 was also weighed and dissolved in 175 µL of acetone and the solution was mixed with the dioxane solution. To this mixture, 5mg PGE₁ dissolved in 250 µL of acetone was added followed by a predetermined amount of diethanolamine dissolved in 100 µL of acetone. A predetermined amount of a 500mM anhydrous ferric chloride solution in acetone was immediately added and the mixture was thoroughly mixed. The mixture was then left at room temperature for 10 minutes. To 25 mL of water placed in a 50mL, sample vial and being stirred with a 2cm stirrer bar, the reaction mixture was slowly added dropwise using a 3mL syringe fitted with a 26G injection needle (stirrer rotation speed = 1000 rpm, injection needle = 26G, syringe = 3 mL Nipro, addition rate = 48 L/hr). To the resulting suspension, 2.5 mL of a 500mM aqueous EDTA solution (pH 7) and 12 µL of an aqueous solution of 200mg/mL Tween 80 (polyoxyethylene (20) sorbitan monooleate) were added. The mixture was concentrated by ultrafiltration (membrane YM-50, manufactured by Amicon), followed by the addition of a 50mM aqueous EDTA solution (pH 7) and concentration (This was repeated twice). Subsequently, the concentrated suspension was sonicated for 30 seconds and was centrifuged (1000 rpm, 5 min) to remove aggregates. The suspension was then analyzed for the particle size on a dynamic light scattering analyzer. The amount of encapsulated PGE₁ was also quantified by HPLC.

The relationship between the amount of PGE₁ encapsulated by the nanoparticles (i.e., encapsulation efficiency) and the amounts of ferric chloride and diethanolamine used was shown in Table 1. The relationship between the encapsulation efficiency of PGE₁ by the nanoparticles and the amount of ferric chloride added was shown in Table 2. The relationship between the encapsulation efficiency of PGE₁ and the particle size of the nanoparticles at different mixing ratios of PDLLA-PEG and PLLA is shown in Table 3.

Table 1: Encapsulation efficiency of PGE₁ by nanoparticles prepared using a constant amount of iron (7.5 µmol) with varying amounts of diethanolamine.

**[Table 1]**

| | | | | | | | | |
|---|---|---|---|---|---|---|---|---|
| Diethanolamine/iron (molar ratio) | 0.0 | 1.0 | 2.0 | 3.0 | 4.0 | 6.0 | 8.0 | 10.0 |
| Encapsulation efficiency of PGE₁ by nanoparticles (wt%) | 0.15 | 0.39 | 0.52 | 0.77 | 1.22 | 1.23 | 1.04 4 | 0.94 |

The results of Table 1 indicate that the encapsulation efficiency of PGE₁ is influenced by the amount of diethanolamine: the encapsulation efficiency was maximized at an optimal amount of diethanol amine.

Table 2: Encapsulation efficiency of PGE₁ by nanoparticles prepared by varying the total amount of iron

**[Table 2]**

| | | | | | | |
|---|---|---|---|---|---|---|
| Amount of iron used (µmol) | 0.0 | 0.5 | 1.5 | 3 | 5 | 7.5 |
| Encapsulation efficiency of PGE₁ by nanoparticles (wt%) | 0.20 | 0.43 | 0.85 | 1.01 | 1.22 | 1.24 |

The results of Table 2 indicate that the encapsulation efficiency of PGE₁ is influenced by the amount of iron added: the encapsulation efficiency increased with increasing amounts of iron.

Table 3: The particle size and encapsulation efficiency of PGE₁ by nanoparticles prepared by varying the mixing ratio of PDLLA-PEG to PLLA

**[Table 3]**

| | | | | | | | |
|---|---|---|---|---|---|---|---|
| The proportion of PEG in PDLLA-PEG/PLLA mixture (wt%) | 5 | 10 | 20 | 30 | 40 | 50 | 64 |
| Particle size (nm) | 203 | 180 | 158 | 116 | 79 | 46 | 24 |
| Encapsulation efficiency of PGE₁ by nanoparticles (wt%) | 2.79 | 2.09 | 1.63 | 1.23 | 0.70 | 0.12 | 0.01 |

The results of Table 3 indicate that smaller particles are formed and a lower encapsulation efficiency of PGE₁ results at a lower mixing ratio of PDLLA-PEG to PLLA.
In each of the nanoparticles shown in Tables 1 to 3 above, the crystallization of PLLA in water was avoided and the stable particles with a lasting particle size were obtained by using PDLLA-PEG.

### Example 3: Sustained release of nanoparticles formed of PLLA and PDLLA-PEG and encapsulating prostaglandin E₁ (PGE₁)

Nanoparticles formed of PLLA and PDLLA-PEG and encapsulating PGE₁ (referred to as PLLA particles) were prepared in the same manner as in Example 2, except that 5 mg of PGE₁, 13 mg of PLLA, 12 mg of PDLLA-PEG, 4.7 mg of diethanolamine and 7.5 µmol of ferric chloride were used.
As a control, nanoparticles formed of PDLLA and PDLLA-PEG and encapsulating PGE₁ (referred to as PDLLA particles) were prepared by using poly DL-lactic acid (PDLLA), the DL-form, in place of PLEA.

The PLLA particles or the PDLLA particles so obtained were dispersed in an equal mixture of phosphate buffered saline and bovine serum and were incubated at 37°C. As a measure of the sustained release properties of both particles, the amount of PGE₁ remaining in the particles were determined by HPLC over time.
The results are shown in Table 4.

Table 4: Release from PGE₁ nanoparticles (Sustained release property)

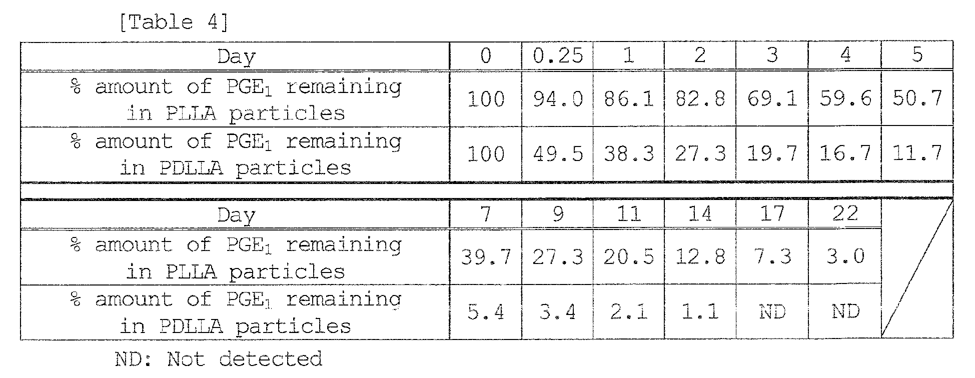

The results of Table 4 indicate that the nanoparticles formed of PLLA (L-form) are capable of releasing PGE₁ more slowly over a longer time period than the nanoparticles formed of PDLLA (DL-form) and are therefore proved to have more favorable sustained release properties.

### Example 4: Cellular uptake of nanoparticles formed of PLLA and PDLLA-PEG and encapsulating prostaglandin E₁ (PGE₁)

Nanoparticles encapsulating fluorescent dye rhodamine were prepared in the same manner as in Example 3, except that rhodamine was added to the acetone solution. As a control, nanoparticles formed of PLLA alone and encapsulating rhodamine and PGE₁ were prepared without using PDLLA-PEG. Each of the two types of nanoparticles was added to murine macrophage-like cells (RAW cells) and incubated at 37°C for 2 hours. Subsequently, the cells were observed by a fluorescence microscope.

The results are shown in Fig. 1. As can be clearly seen from Fig. 1, the control nanoparticles formed of PLLA alone were significantly incorporated by cells, whereas the cellular incorporation of nanoparticles formed of PLLA and PDLLA-PEG was suppressed.

In Fig. 1, nuclei were stained blue with DAPI (4',6-diamino-2-phenylindole) and rhodamine distribution was stained red. Picture A) shows the density distribution for cells with rhodamine alone, Picture B) shows the density distribution with the nanoparticles made from PLLA alone encapsulating rhodamine and PGE₁, and Picture C) shows the density distribution with nanoparticles made from PLLA and PDLLA-PEG encapsulating rhodamine and PGE₁. The scale bar indicates 50 µm.

### Example 5: Production of nanoparticles formed of PLLA and PDLLA-PEG and encapsulating betamethasone phosphate

36 mg of PLLA (manufactured by Taki Chemical Co., Ltd.) was weighed and dissolved in 450 µl of dioxane. 14 mg of PLLA-PEG synthesized in Example 1 and 7.5 mg of diethanolamine were also weighed and dissolved in 1050 µL of acetone and the solution was mixed with the dioxane solution. To this mixture, 68 µL of a 1M aqueous zinc chloride solution was added, followed by 28 µL of an aqueous solution of 350mg/mL betamethasone phosphate. The mixture was allowed to stand at room temperature for approx. 30 minutes. To 25 mL of water placed in a 50mL sample vial and being stirred with a 2cm stirrer bar, the reaction mixture was slowly added dropwise using a 3mL syringe fitted with a 26G injection needle (stirrer rotation speed = 1000 rpm, injection needle = 26G, syringe = 3 mL Nipro, addition rate = 48 L/hr). To the resulting suspension, 500 µL of a 0.5M aqueous sodium citrate solution (pH 7) and 125 µL of an aqueous solution of Tween 80 (polyoxyethylene (20) sorbitan monooleate) (200 mg/mL) were added. The mixture was concentrated by ultrafiltration (membrane YM-50, manufactured by Amicon), followed by the addition of water and concentration (This was repeated twice). Subsequently, the concentrated suspension was sonicated for 30 seconds and was centrifuged (1000 rpm, 5 min) to remove aggregates.

The particle size of the resulting nanoparticles was measured on a dynamic light scattering analyzer. The encapsulation efficiency of betamethasone phosphate by the nanoparticles was also quantified by HPLC.

As a result, nanoparticles formed of PLLA and PDLLA-PEG and encapsulating betamethasone phosphate were obtained. The encapsulation efficiency of betamethasone phosphate by the nanoparticles was 8.6 wt%. The particle size was determined to be about 100 nm.

### Example 6: Production of nanoparticle formed of PLLA and PLLA-PEG and encapsulating prednisolone phosphate

As in Example 5, PLLA (manufactured by Taki Chemical Co., Ltd.), PDLLA-PEG synthesized in Example 1 and diethanolamine were dissolved in acetone and the solution was mixed with a PLLA solution in dioxane. To this mixture, a 1M aqueous zinc chloride solution was added, followed by an aqueous solution of prednisolone phosphate.
The mixture was then treated in the same manner as in Example 5 to obtain nanoparticles encapsulating prednisolone phosphate.
The particle size of the resulting nanoparticles was measured on a dynamic light scattering analyzer and the encapsulation efficiency of prednisolone phosphate by the nanoparticle was also quantified by HPLC. As a result, nanoparticles formed of PLLA and PDLLA-PEG and encapsulating prednisolone phosphate were obtained. The encapsulation efficiency was 8.5 wt% and the particle size was determined to be about 100 nm.

### INDUSTRIAL APPLICABILITY

As set forth, the nanoparticle containing a low-molecular-weight drug having a negatively charged group of the present invention can effectively target the low-molecular-weight drug having a negatively charged group to an affected site, is capable of sufficiently sustained release of the drug, has a reduced tendency to accumulate in the liver to cause reduced side effects, and has an improved long-circulating property. The nanoparticle of the present invention is therefore useful as a pharmaceutical product and is of significant medical importance.

## Claims

1. A nanoparticle containing a low-molecular-weight drug having a negatively charged group, the nanoparticle obtained by hydrophobicizing a low-molecular-weight drug having a negatively charged group with a metal ion, and reacting the hydrophobicized drug with poly L-lactic acid or poly(L-lactic acid/glycolic acid) copolymer and poly DL- or L-lactic acid-polyethylene glycol block copolymer or poly(DL- or L-lactic acid/glycolic acid)-polyethylene glycol block copolymer.

2. The nanoparticle containing a low-molecular-weight drug having a negatively charged group according to claim 1, further containing a basic low-molecular-weight compound mixed therewith.

3. The nanoparticle containing a low-molecular-weight drug having a negatively charged group according to claim 1 or 2, further containing a surfactant compounded thereinto.

4. The nanoparticle containing a low-molecular-weight drug having a negatively charged group according to any of claims 1 to 3, having a particle size of 20 to 300 nm, and preferably 50 to 200 nm.

5. The nanoparticle containing a low-molecular-weight drug having a negatively charged group according to claim 1 or 2, wherein the metal ion is one or two or more of zinc ion, iron ion, copper ion, nickel ion, beryllium ion, manganese ion or cobalt ion.

6. The nanoparticle containing a low-molecular-weight drug having a negatively charged group according to claim 1 or 2, wherein the low-molecular-weight drug having the negatively charged group has a phosphate group, a sulfate group or a carboxyl group due to the hydrophobicization with the metal ion.

7. The nanoparticle containing a low-molecular-weight drug having a negatively charged group according to claim 1, 2 or 6, wherein the low-molecular-weight drug having the negatively charged group is an anti-inflammatory steroid, a non-steroid anti-inflammatory agent, prostaglandin or a derivatives thereof, an antimicrobial agent or an anticancer agent.

8. The nanoparticle containing a low-molecular-weight drug having a negatively charged group according to claim 1 or 2, wherein the poly DL- or L-lactic acid-polyethylene glycol block copolymer or poly(DL- or L-lactic acid/glycolic acid)-polyethylene glycol block copolymer has a weight average molecular weight of 3,000 to 30,000.

9. The nanoparticle containing a low-molecular-weight drug having a negatively charged group according to claim 2, wherein the basic low-molecular-weight compound is one or two or more selected from (dimethylamino)pyridine, pyridine, piperidine, pyrimidine, pyrazine, pyridazine, quinoline, quinuclidine, isoquinoline, bis (dimethylamino)naphthalene, naphthylamine, morpholine, amantadine, aniline, spermine, spermidine, hexamethylenediamine, putrescine, cadaverine, phenetylamine, histamine, diazabicyclooctane, diisopropylethylamine, monoethanolamine, diethanolamine, triethanolamine, ethylamine, diethylamine, triethylamine, methylamine, dimethylamine, trimethylamine, triethylenediamine, diethylenetriamine, ethylenediamine and trimethylenediamine.

10. The nanoparticle containing a low-molecular-weight drug having a negatively charged group according to claim 3, wherein the surfactant is one or two or more selected from phosphatidylcholine, polyoxyethylene (20) sorbitan monooleate, polyoxyethylene (20) sorbitan monolaurate, polyoxyethylene (20) sorbitan monostearate, polyoxyethylene (20) sorbitan monopalmitate, polyoxyethylene (20) sorbitan trioleate, polyoxyethylene (80) octylphenyl ether, polyoxyethylene (20) cholesterol ester, lipid-polyethylene glycol, polyoxyethylene hydrogenated castor oil and fatty acid-polyethylene glycol copolymer.

11. A parenteral preparation containing as an active ingredient the nanoparticle containing a low-molecular-weight drug having a negatively charged group according to claims 1 to 10.

12. The parenteral preparation according to claim 11, wherein the preparation is an intravenous preparation, a local injection preparation, a nasal preparation, an ophthalmic preparation, an inhalation preparation or a spray preparation.

13. A method for producing the nanoparticle containing a low-molecular-weight drug having a negatively charged group according to claim 1, the method comprising:
mixing the low-molecular-weight drug having the negatively charged group with the metal ion in a solvent to obtain the hydrophobicized drug; and
mixing with the resulting mixture the poly L-lactic acid or the poly(L-lactic acid/glycolic acid) copolymer and the poly DL- or L-lactic acid-polyethylene glycol block copolymer or the poly(DL- or L-lactic acid/glycolic acid)-polyethylene glycol block copolymer.

14. The method according to claim 13, for producing the nanoparticle containing a low-molecular-weight drug having a negatively charged group, the method further comprising mixing a basic low-molecular-weight compound therewith.

15. The method according to claim 14, for producing the nanoparticle containing a low-molecular-weight drug having a negatively charged group, wherein the basic low-molecular-weight compound is one or two or more selected from (dimethylamino)pyridine, pyridine, piperidine, pyrimidine, pyrazine, pyridazine, quinoline, quinuclidine, isoquinoline, bis(dimethylamino)naphthalene, naphthylamine, morpholine, amantadine, aniline, spermine, spermidine, hexamethylenediamine, putrescine, cadaverine, phenetylamine, histamine, diazabicyclooctane, diisopropylethylamine, monoethanolamine, diethanolamine, triethanolamine, ethylamine, diethylamine, triethylamine, methylamine, dimethylamine, trimethylamine, triethylenediamine, diethylenetriamine, ethylenediamine and trimethylenediamine.
